# EUROPÄISCHE PATENTSCHRIFT

(11) **EP 3 930 777 B1**
(45) Veröffentlichungstag und Bekanntmachung des Hinweises auf die Patenterteilung: **16.08.2023**
(21) Anmeldenummer: 20706223.3
(22) Anmeldetag: 19.02.2020
(51) Int. Cl.: A61L 31/02, A61L 31/08, A61L 31/14, A61L 31/18

(54) **INTRAVASKULÄRES FUNKTIONSELEMENT, SYSTEM MIT EINEM FUNKTIONSELEMENT UND VERFAHREN**
INTRAVASCULAR FUNCTIONAL ELEMENT, SYSTEM HAVING A FUNCTIONAL ELEMENT, AND METHOD
ÉLÉMENT FONCTIONNEL INTRAVASCULAIRE, SYSTÈME COMPRENANT UN ÉLÉMENT FONCTIONNEL, ET PROCÉDÉ

(30) Priorität: 26.02.2019 DE 102019104827
(43) Veröffentlichungstag der Anmeldung: 05.01.2022
(73) Patentinhaber: Acandis GmbH, 75177 Pforzheim (DE)
(72) Erfinder: CATTANEO, Giorgio, 76199 Karlsruhe (DE); SCHÜSSLER, Andreas, 76327 Pfinztal (DE); STROBEL, Martin, 75177 Pforzheim (DE); JANISCH, Christoph, 75305 Neuenbürg (DE)
(74) Vertreter: Meissner Bolte Partnerschaft mbB
(86) Internationale Anmeldenummer: PCT/EP2020/054285
(87) Internationale Veröffentlichungsnummer: WO 2020/173764

(56) Entgegenhaltungen:
- EP-A1- 1 522 605
- EP-A1- 2 765 216
- EP-A2- 2 764 877
- WO-A1-2010/124050
- WO-A1-2012/038522
- WO-A2-2008/019116
- DE-A1-102016 107 791

## Beschreibung

Die Erfindung betrifft ein intravaskuläres Funktionselement, ein System mit einem solchen Funktionselement und ein Verfahren.

In der Medizintechnik werden Stents in der Regel durch Laserverfahren hergestellt. Es kommen aber auch Geflechte aus Nitinol-Drähten für Implantate (z.B. Stents oder Okkluder) zum Einsatz. Anders als bei durch Laserverfahren hergestellten Stents, gleiten bei Drahtgeflechten die Drähte übereinander und ermöglichen daher eine gute Verformung der Stent-Strukturen. Grundsätzlich können (vaskuläre) Implantate aus Halbzeugen, wie Blechen, Präzisionsrohren oder Drähten gefertigt werden.

Beispielsweise beschreibt US 2004/0117001 A1 ein Verfahren zur Herstellung eines Stents aus Nitinol. Ein Ziel der US 2004/0117001 A1 besteht darin, den Nickel-Gehalt in einer oberflächennahen Schicht zu reduzieren, um zu verhindern, dass Nickel aus der Schicht freigesetzt wird, da dadurch die Biokompatibilität des Stents beeinträchtigt wird. Zur Herstellung der Stents wird ein Laser-Verfahren vorgeschlagen. Nach einem Kaltbearbeitungsschritt wird der Stent wärmebehandelt und dann bei Temperaturen unter 20°C elektropoliert. Zur thermischen Oxidierung wird der Stent mit Heißdampf bei einer Temperatur von 150°C für 12h beaufschlagt. Dadurch soll eine oxidische Oberfläche mit einem Ni-Gehalt von weniger als 2 Gew.% in einer Schichttiefe von 10 nm erreichbar sein.

Das bekannte Verfahren hat den Nachteil, dass die damit herstellbaren Oxidschicht bei Implantaten, die aus Drahtgeflechten bestehen, schnell verschleißen.

EP 2 765 216 A1, die auf die Anmelderin zurückgeht, beschreibt ein Herstellungsverfahren, bei dem durch eine Wärmebehandlung in einem speziellen Salzbad das vorstehend genannte Problem gelöst wird. Das mittlerweile patentierte Herstellungsverfahren ist unter dem Markennamen BlueOxide bekannt und wird zur Oberflächenbehandlung geflochtener Stents oder geflochtener Flow-Diverter, beispielsweise bei dem unter dem Markennamen DERIVO von der Anmelderin hergestellten Flow-Diverter, eingesetzt. Aus EP 2 764 877 A2, die ebenfalls auf die Anmelderin zurückgeht, ist ein weiteres Funktionselement mit einer oxidierten Oberfläche bekannt. WO 2010/124 050 A1 zeigt einen Stent, der eine Beschichtung aufweist, welche Titanoxid umfasst.

Implantate sollen biokompatibel, also im Körper möglichst verträglich sein und gute mechanische Eigenschaften aufweisen. Für eine gute Biokompatibilität ist es bspw. günstig, wenn möglichst geringe Mengen an Elementen aus der Legierungszusammensetzung des Implantats, bspw. Nickel aus der Gitterstruktur freigesetzt und in die Blutbahn gelangen können. Außerdem soll das Implantat möglichst gut komprimierbar und im implantierten Zustand im Gefäß möglichst compliant sein. An dieser Stelle setzt die Erfindung ein, deren Aufgabe es ist, ein intravaskuläres Funktionselement mit guten biokompatiblen und mechanischen Eigenschaften anzugeben. Eine weitere Aufgabe der Erfindung ist es, ein System mit einem solchen Funktionselement sowie ein Verfahren zur Herstellung eines entsprechenden Funktionselements anzugeben.

Diese Aufgabe wird im Hinblick auf das Funktionselement durch den Gegenstand des Anspruches 1, im Hinblick auf das System durch den Gegenstand des Anspruch 13 und im Hinblick auf das Verfahren durch den Gegenstand des Anspruchs 14 gelöst.

Insbesondere wird die Aufgabe durch ein intravaskuläres Funktionselement mit einer zumindest abschnittsweise rohrförmigen, radial selbstexpandierbaren Gitterstruktur, die einen Draht oder mehrere Drähte aufweist, wobei der Draht / wenigstens einer der Drähte ein superelastisches Material, insbesondere ein superelastisches Material aus einer Legierung mit den Legierungselementen Nickel und Titan, umfasst. Bei dem Funktionselement kann es sich um ein Implantat, insbesondere Stent, Flow-Diverter, Stent-Graft und intravaskuläres Verschlussdevice handeln. Das Funktionselement zeichnet sich dadurch aus, dass die Mischoxidschicht auf der Oberfläche des Drahtes / der Drähte mit einer Schichtdicke von 250 nm bis 400 nm ausgebildet ist.

Die Erfindung hat verschiedene Vorteile. Überraschenderweise hat sich gezeigt, dass eine sehr dünne Mischoxidschicht besonders wirksam die Diffusion von Legierungselementen in den Körper verringert. Man nimmt an, dass die Schichtdicke im beanspruchten Bereich zu einer sehr dichten und im Vergleich zu dickeren Schichten kompakten, d.h. weniger porösen Schicht führt und eine besonders wirksame Diffusionsbarriere bildet. Außerdem weist die Oberfläche des erfindungsgemäßen Funktionselements auf Grund der guten Haftung der Mischoxidschicht auf der Drahtoberfläche gute mechanische Eigenschaften auf. Ein weiterer Vorteil besteht darin, dass zur Bildung der Mischoxidschicht andere Verfahren zur Oberflächenbehandlung als das übliche Elektropolieren zum Einsatz kommen können, bspw. mechanisches Polieren, wodurch die Kosten reduziert werden können.

Besondere Ausführungsformen der Erfindung sind in den Unteransprüchen angegeben.

Erfindungsgemäß beträgt die quadratische Rauheit R_{q} des Drahtes / der Drähte von 0,02 µm bis 0,5 µm, insbesondere von 0,05 µm bis 0,2 µm, insbesondere von 0,06 µm bis 0,1 µm.

Die quadratische Rauheit R_{q} (auf Englisch rms-roughness oder root-mean-squared roughness: Wurzel des Mittelquadrates) wird in an sich bekannter Weise aus dem Mittel der Abweichungsquadrate berechnet und entspricht dem "quadratischen Mittel". Zur Rauheitsmessung kann bspw. die an sich bekannte Weißlichtinterferometrie (WLI) eingesetzt werden, die als berührungslose optische Messmethode, die Interferenz breitbandigen Lichts (Weißlicht) ausnutzt und so 3D-Profilmessungen erlaubt. Es ist auch möglich 3D-Laserscanning-Mikroskopie einzusetzen, mit der eine ausreichende Auflösung möglich ist.

Die im Vergleich zu dem bekannten Verfahren nach EP 2 765 216 A1 erhöhte Rauheit R_{q} des Drahtes / der Drähte von 0,02 µm bis 0,05 µm erlaubt die Anwendung anderer Herstellungsverfahren als das bekannte Elektropolieren, die mit Blick auf die angestrebten homogenen Eigenschaften des zu Herstellung des Funktionselements verwendeten Drahtes bzw. der zur Herstellung des Funktionselements verwendeten Drähte optimiert sind. Beispielsweise können solche Herstellungsverfahren, insbesondere mechanisches Polieren, verwendet werden, die zu einem optimierten Drahtdurchmesser führen, der positiven Einfluss auf die Crimpeigenschaften des Funktionselements hat.

Erfindungsgemäß beträgt die quadratische Rauheit R_{q} des Drahtes / der Drähte von 0,02 µm bis 0,5 µm, insbesondere von 0,05 µm bis 0,2 µm, insbesondere von 0,06 µm bis 0,1 µm . Diese Bereiche haben sich als besonders vorteilhaft sowohl mit Blick auf die Verschleißeigenschaften als auch mit Blick auf die Einstellung homogener Eigenschaften des für die Gitterstruktur wendeten Drahtes bzw. der für die Gitterstruktur verwendeten Drähte erwiesen.

Alternativ oder zusätzlich ist bei der Erfindung vorgesehen, dass die Rauheit, insbesondere die quadratische Rauheit R_{q} in Umfangsrichtung des Drahtes / der Drähte größer als die Rauheit, insbesondere die quadratische Rauheit R_{q} in Längsrichtung des Drahtes / der Drähte ist. Dieses anisotrope Rauheitsprofil verbessert die Transporteigenschaften des Funktionselements beim Implantieren und kann bspw. dadurch eingestellt werden, dass das anisotrope Rauheitsprofil beibehalten wird, das sich durch die Herstellung der Drähte durch Drahtziehen einstellt. Dies kann bspw. dadurch erreicht werden, dass die gezogenen Drähte so oberflächenbehandelt werden, dass eine größere Rauheit als durch das Elektropolieren eingestellt wird und die gewünschte Anisotropie erhalten bleibt. Die Oberflächenbehandlung umfasst Deoxidieren und/oder mechanisches Polieren und/oder chemisches Polieren. Das mechanische Polieren umfasst bspw. Schleifen. Das chemische Polieren umfasst bspw. Beizen. Unter Deoxidation wird die Verringerung der Oxidschichtdicke bzw. die Änderung der Zusammensetzung und/oder Eigenschaften der Oxidschicht verstanden.

Allgemein wird der Draht bzw. werden die Drähte nicht elektropoliert.

Vorzugsweise ist die Rauheit in Umfangsrichtung mindestens um den Faktor 1,5, insbesondere mindestens um den Faktor 2, insbesondere mindestens um den Faktor 5, insbesondere mindestens um den Faktor 10 größer als die Rauheit in Längsrichtung. Die quadratische Rauheit R_{q} in Umfangsrichtung des Drahtes / der Drähte kann von 0,1 µm bis 0,5 µm betragen. In Längsrichtung des Drahtes / der Drähte kann die quadratische Rauheit R_{q} von 0,02 µm bis 0,1 µm, insbesondere von 0,05 µm bis 0,08 µm betragen.

Bei einer besonders bevorzugten Ausführungsform ist der Durchmesser des Drahtes / der Drähte entlang der gesamten Drahtlänge im Wesentlichen konstant. Besonders vorteilhaft weicht der Durchmesser des Drahtes / der Drähte um höchstens 10%, insbesondere höchstens 5%, insbesondere höchstens 3%, insbesondere höchstens 2%, insbesondere höchstens 1% vom durchschnittlichen Durchmesser des Drahtes / des jeweiligen Drahtes ab. Der im Wesentlichen konstante Durchmesser des Drahtes/der Drähte hat den Vorteil, dass dadurch die mechanischen Eigenschaften des Funktionselements gleichmäßig und homogen entlang der Gitterstruktur ausgebildet sind. Mit anderen Worten sind die mechanischen Eigenschaften des Funktionselements entlang der Längsachse der Gitterstruktur und in Umfangsrichtung der Gitterstruktur gleichmäßig und homogen. Dadurch wird beispielsweise das Crimpverhalten des Funktionselements verbessert. Andere Eigenschaften, bei denen der Drahtdurchmesser eine Rolle spielt, werden ebenfalls verbessert.

Wenn der Drahtdurchmesser um höchstens 10 %, insbesondere höchstens 5 %, insbesondere höchstens 3 %, insbesondere höchstens 2%, insbesondere höchstens 1% vom durchschnittlichen Durchmesser des Drahtes / des jeweiligen Drahtes abweicht, wird eine besonders gute Verbesserung der Homogenität der Eigenschaften der Gitterstruktur erreicht.

Wenn der durchschnittliche Durchmesser des Drahtes / der Drähte von 30 µm bis 60 µm, insbesondere von 40 µm bis 60 µm beträgt, kann ein breites Spektrum von Anwendungsfällen für das intravaskulären Funktionselement abgedeckt werden. Dasselbe gilt für den Nenndurchmesser der Gitterstruktur von 2,5 mm bis 8 mm, insbesondere von 3 mm bis 5,5 mm, insbesondere ca. 3,5 mm oder insbesondere ca. 4,5 mm.

Der Nenndurchmesser der Gitterstruktur entspricht im Wesentlichen dem maximalen Einsatzdurchmesser, d. h. dem vom Hersteller des Funktionselement empfohlenen maximalen Durchmesser (Intended Use Bereich). Der Unterschied zwischen dem vom Hersteller des Funktionselement empfohlenen maximalen Durchmesser und dem Ruhedurchmesser, bei dem keine äußeren Kräfte auf das Funktionselement wirken, besteht in der Wandstärke des Funktionselements. Beispielsweise beträgt der Ruhedurchmesser eines Funktionselements, insbesondere eines Stents oder eines Flow-Diverters ca. 4,7 mm bei einem Nenndurchmesser von 4,5 mm und einer Drahtstärke von 50 µm. Der Unterschied ergibt sich aus der vierfachen Drahtstärke, da bei einer geflochtenen Gitterstruktur jeweils zwei übereinander angeordnete Drähte zu berücksichtigen sind.

Zur Verbesserung der Röntgensichtbarkeit bei weitgehender Beibehaltung der mechanischen Eigenschaften kann vorgesehen sein, dass der Draht / wenigstens einer der Drähte ein röntgensichtbares Kernmaterial und ein superelastisches Mantelmaterial aufweist.

Bei einer weiteren besonders bevorzugten Ausführungsform ist der Draht oberflächenbehandelt, insbesondere deoxidiert, insbesondere mechanische poliert und/oder chemisch poliert, insbesondere gebeizt. Wenn die Gitterstruktur aus mehreren Drähten besteht, sind die Drähte jeweils oberflächenbehandelt, insbesondere deoxidiert, insbesondere mechanische poliert und/oder chemisch poliert, insbesondere gebeizt. Wenn die Drähte bspw. durch Ziehen hergestellt sind, ist es möglich, durch die vorstehende Oberflächenbehandlung die gewünschte Rauheit einzustellen. Die Oberflächenbehandlung ermöglicht eine größere Rauheit als durch die übliche Elektropolitur. Es hat sich zudem herausgestellt, dass der Drahtdurchmesser entlang seiner Länge im Wesentlichen konstant ist, wenn der Draht bei der Herstellung wie vorstehend erläutert oberflächenbehandelt wird. Jedenfalls können dadurch besonders gute homogene Eigenschaften des Funktionselements erreicht werden.

Die auf der Drahtoberfläche gebildete Oxidschicht ist nickelarm und enthält TiOz, wodurch das Korrosionsverhalten und die Biokompatibilität des Funktionselements verbessert werden. Die Ausbildung der Oxidschicht als Mischoxidschicht, in der wenigstens Titanitrid und/oder Titanoxinitrid vorhanden ist, erhöht die Schichthärte, wodurch der Verschleiß bei Beanspruchung des Funktionselementes, insbesondere des Implantats, im Gefäß verringert wird. Dieser Vorteil kommt besonders bei Geflechten, wie bei geflochtenen Stents zum Tragen, bei denen Drähte sich berühren und aufeinander gleiten. Damit wird die Qualität des Funktionselementes, insbesondere des Implantats verbessert, bspw. mit Blick auf die Compliance im Gefäß. Überdies wird der Reibungskoeffizient der Oberfläche des Funktionselementes, insbesondere des Implantats verringert, was zu einem verbesserten Gleitverhalten im Katheter führt. Die guten Gleiteigenschaften wirken einerseits zwischen den Drähten selbst, wodurch die Crimpbarkeit, d.h. die Fähigkeit des Funktionselementes komprimiert zu werden, verbessert wird. Andererseits wirken die guten Gleiteigenschaften zwischen den Drähten und der Katheterinnenwand.

Die dadurch verringerte Schiebekraft, die zum Bewegen des Funktionselements, insbesondere Implantats im Katheter erforderlich ist, erhöht die Sicherheit, da das Risiko des Blockierens und der Beschädigung des Funktionselements, insbesondere Implantats im Katheter verringert wird. Gleiches gilt für Zuführsysteme, bei denen die Zuführung des Funktionselements nicht durch Bewegung des Funktionselements selbst sondern durch eine Relativbewegung zwischen einem Teil des Zuführsystems und dem Funktionselement bewirkt wird.

Die Ausführungsform, bei der die Gitterstruktur in Längsrichtung und in Umfangsrichtung jeweils Zellen aus sich kreuzenden Drähten bildet und in Umfangsrichtung 16 Zellen bis 32 Zellen, insbesondere 20 Zellen bis 28 Zellen, insbesondere 24 Zellen aufweist, wobei die Gitterstruktur Schlaufen an einem einzigen axialen Ende aufweist, ist besonders gut für Flow-Diverter geeignet, bei denen es darauf ankommt, dass die Porosität des Funktionselements so eingestellt ist, dass der Blutstrom im Gefäß geführt wird, beispielsweise zur Behandlung von Aneurysmen. Ein Beispiel für einen Flow-Diverter, auf den das vorstehend genannte Ausführungsbeispiel anwendbar ist, ist der unter dem Markennamen DERIVO bekannte Flow-Diverter der Anmelderin. Die Schlaufen bestehen jeweils aus einem umgelenkten Draht.

Die in den Unteransprüchen 9, 10 angegebenen Konstruktionsmerkmale führen zu einer Verbesserung der homogenen Eigenschaften bzw. der Röntgensichtbarkeit des im Zusammenhang mit einem als Flow-Diverter ausgebildeten Funktionselement.

Die Ausführungsform, bei der die Gitterstruktur in Längsrichtung und in Umfangsrichtung jeweils Zellen aus einem einzigen mit sich selbst verflochtenen Draht bildet und in Umfangsrichtung 6 Zellen bis 16 Zellen, insbesondere 8 Zellen bis 12

Zellen aufweist, wobei die Gitterstruktur Schlaufen an beiden axialen Enden aufweist, ist für ein als geflochtener Stent ausgebildetes Funktionselement geeignet, bspw. für den unter dem Markennamen ACCERO von der Anmelderin hergestellten Stent.

Die in den Unteransprüchen 11, 12 angegebenen Konstruktionsmerkmale führen zu einer Verbesserung der homogenen Eigenschaften bzw. der Röntgensichtbarkeit des im Zusammenhang mit einem als Stent ausgebildeten Funktionselements.

Die Anzahl der Zellen auf dem Umfang wird durch die Anzahl der Drähte oder die Anzahl der Drahtsegmente bestimmt, die die Gitterstruktur bilden. Die Anzahl der Drähte oder die Anzahl der Drahtsegmente wird dadurch ermittelt, dass eine imaginäre Schnittfläche die Gitterstruktur senkrecht zu deren Längsachse schneidet. Die Schnittfläche verläuft also in radialer Richtung bezogen auf die röhrchenförmige Gitterstruktur. Die Anzahl der Drahtschnittpunkte in der Schnittfläche entspricht der Anzahl der Drähte, insbesondere Einzeldrähte, bzw. der Anzahl der Drahtsegmente der Gitterstruktur. Allgemein sind doppelt so viele Drähte oder Drahtsegmente wie Zellen vorhanden, wobei eine erste Hälfte der Drähte oder Drahtsegmente in einer ersten Spiralrichtung und eine zweite Hälfte der Drähte oder Drahtsegmente in einer zweiten Spiralrichtung entgegengesetzt zur ersten Spiralrichtung verlaufen, so dass sich die Drähte oder Drahtsegmente kreuzen und Zellen bilden.

Bspw. werden 6 Zellen auf dem Umfang durch 12 Drähte oder Drahtsegmente gebildet, wobei 6 Drähte oder Drahtsegmente im Uhrzeigersinn und 6 Drähte oder Drahtsegmente im Gegenuhrzeigersinn umlaufen.

Bei einem Funktionselement mit einer geflochtenen Gitterstruktur werden die Zellen auch als Maschen bezeichnet.

Die Gitterstruktur kann aus mehreren Einzeldrähten gebildet sein, die ohne Umlenkung an den beiden axialen Enden der Gitterstruktur, d.h. jeweils mit freien, bzw. offenen Drahtenden verlaufen und zur Bildung der Zellen sich kreuzen, insbesondere verflochten sind. Es ist auch möglich, dass die Gitterstruktur aus einem einzigen Einzeldraht gebildet ist, der an beiden axialen Enden der Gitterstruktur umgelenkt ist und dort jeweils Schlaufen bildet. Die Zellen sind durch Drahtsegmente oder Drahtabschnitte des Einzeldrahtes gebildet, die in unterschiedlichen Spiralrichtungen auf dem Umfang des Funktionselements verlaufen und zur Bildung der Zellen sich kreuzen, insbesondere verflochten sind. Ferner kann die Gitterstruktur aus mehreren Einzeldrähten gebildet sein, die zumindest an einem axialen Ende der Gitterstruktur umgelenkt sind und sich kreuzende, insbesondere verflochtene Drahtsegmente bilden. Damit kann dieselbe Zellenanzahl auf dem Umfang bei verschiedenen Funktionselementen durch unterschiedlich viele Einzeldrähte gebildet sein, je nachdem, wie viele Einzeldrähte an einem oder an beiden axialen Enden der Gitterstruktur umgelenkt sind. Wenn die Gitterstruktur durch einen einzigen, mehrfach umgelenkten Einzeldraht gebildet ist, sollte die Anzahl der Zellen auf dem Umfang auf maximal 16 Zellen beschränkt sein, da die Herstellung der Gitterstruktur sonst sehr zeitaufwändig ist.

Der Winkel o, insbesondere der Flechtwinkel o, beträgt zumindest abschnittsweise ≥ 45°, vorzugsweise ≥ 50°, vorzugsweise ≥ 55°, vorzugsweise ≥ 60°, vorzugsweise ≥ 65°, vorzugsweise ≥ 70°, vorzugsweise ≥ 75°, insbesondere maximal 75°. Mit steigendem Flechtwinkel nimmt die Flexibilität des Geflechts zu.

Der Flechtwinkel bezieht sich in diesem Zusammenhang auf den Ruhezustand der des Funktionselements (im expandierten Zustand). Die Bestimmung des Flechtwinkels wird also im entspannten Zustand ohne Einwirkung äußerer Kräfte vorgenommen. Dieser Zustand kann beispielsweise dem Herstellzustand entsprechen. Zur Bestimmung des Flechtwinkels ist das Funktionselement in axialer Richtung geradlinig ausgerichtet. In diesem Zusammenhang wird als Flechtwinkel derjenige Winkel bezeichnet, der zwischen einem Draht und einer senkrechten Projektion der Rotationsachse auf die Umfangsebene der Gitterstruktur bzw. des Geflechts gebildet ist. Die Rotationsachse entspricht der Längsachse des Geflechts bzw. der Gitterstruktur.

Der Drahtquerschnitt ist nicht auf eine bestimmte Form beschränkt. Möglich sind runde, insbesondere kreisrunde, elliptische, eckige oder andere Querschnittsformen des Drahtes. Das Funktionselement kann ein zumindest abschnittsweise, insbesondere vollständig röhrchenförmiges Geflecht, wie einen Stent bilden. Andere Anwendungen, wie Okkluder, Flow Diverter, Filter oder Thrombektomie Devices sind möglich.

Das oben beschriebene Funktionselement, insbesondere Implantat kann Bestandteil eines Systems sein, umfassend das Funktionselement sowie einen Katheter, wobei ein Katheter-Innendurchmesser vorzugsweise ≤ 0,8 mm, weiter vorzugsweise ≤ 0,7 mm, noch weiter vorzugsweise ≤ 0,6 mm, noch weiter vorzugsweise ≤ 0,5 mm, noch weiter vorzugsweise ≤ 0,4 mm, ist. Unabhängig offenbart und beansprucht wird in diesem Zusammenhang auch die Verwendung eines wie oben beschrieben (hergestellten) Funktionselementes, insbesondere Implantates für einen Katheter und/oder als Stent.

Das Drahtgeflecht oder allgemein Drahtgebilde kann zumindest teilweise aus Kompositdrähten hergestellt werden, wobei ein innerer Kern des Drahtes aus einem röntgensichtbaren Material, wie Platin oder Tantal besteht.

AES wird bekanntermaßen zur Analyse der in einer oberflächennahen Schicht enthaltenen Elemente eines Materials eingesetzt. Durch sukzessiven Abtrag der Schicht durch Sputtern wird durch die AES-Analyse der jeweils freigelegten Schichtoberfläche ein Tiefenprofil der Elementverteilung in der Schicht erzeugt, das zur Charakterisierung des Stickstoffgehaltes bezogen auf den Sauerstoffgehalt sowie zum Nachweis des Konzentrationsverlaufs der übrigen Elemente, wie Ni und Ti herangezogen wird. Die gemessene Intensität des jeweiligen Elementes ergibt sich in bekannter Weise aus den bei der AES-Analyse durch Elektronenbeschuss emittierten Auger-Elektronen.

Zur Herstellung des erfindungsgemäßen intravaskulären Funktionselementes, das in ein Hohlorgan einführbar ist, kommt ein Verfahren zum Einsatz, bei dem folgende Schritte durchgeführt werden:
Vor der Herstellung der Gitterstruktur des Funktionselementes werden ein Draht oder mehrere Drähte oberflächenbehandelt oder ein oberflächenbehandelter Draht oder mehrere oberflächenbehandelte Drähte werden bereitgestellt. Die Oberflächenbehandlung wird so durchgeführt, dass die bei der mechanischen Herstellung der Drähte, insbesondere durch Ziehen, sich einstellenden Rauheitswerte weniger stark als bei der bisher üblichen Elektropolitur verringert werden. Dies wird dadurch erreicht, dass die Oberflächenbehandlung ein Deoxidieren und/oder eine mechanische Politur, insbesondere Schleifen, und/oder eine chemische Politur, insbesondere Beizen, der Drahtoberfläche nach der mechanischen Herstellung, insbesondere nach dem Ziehen umfasst. Danach wird die Gitterstruktur aus dem oberflächenbehandelten Draht / den oberflächenbehandelten Drähten gebildet, insbesondere geflochten. Nachdem die Gitterstruktur gebildet, insbesondere geflochten ist, wird eine Oxidschicht auf die Oberfläche des Drahtes / der Drähte mit einer Schichtdicke von 250 nm bis 400 nm durch eine Wärmebehandlung in einem Salzbad aufgebracht.

Für die Bereitstellung eines Drahtmetallkörpers mit metallischer Oberfläche wird eine Vorbehandlung durchgeführt, bei der eine üblicherweise auf der Drahtoberfläche vorhandene Oxidschicht entfernt wird. Diese Oxidschicht mit einer Dicke von 0,2 µm bis 5 µm entsteht bei der Drahtherstellung, wenn zur Einstellung der Materialeigenschaften des Drahtes eine Wärmebehandlung durchgeführt wird. Für die Entfernung der Oxidschicht wird der Draht oberflächenbehandelt, wie vorstehend beschrieben. Wenn die Gitterstruktur mehrere Drähte aufweist, werden vor der Herstellung der Gitterstruktur diese oberflächenbehandelt. Es ist möglich, dass im Rahmen des erfindungsgemäßen Verfahrens die Drähte bzw. der Einzeldraht als ein Teilschritt oberflächenbehandelt werden. Es ist auch möglich, dass in einem gesonderten Verfahren oberflächenbehandelte Drähte bzw. ein oberflächenbehandelter Draht bereitgestellt und im Rahmen des Verfahrens zu dem Funktionselement verarbeitet werden.

Zu den Vorteilen des mit dem erfindungsgemäßen Verfahren hergestellten Funktionselements wird auf die vorstehenden Erläuterungen verwiesen.

Auf der metallischen Oberfläche des Drahtmetallkörpers kann eine erste Oxidschicht ausgebildet werden, auf die die zweite Mischoxidschicht thermisch aufgebracht wird. Die Bildung der ersten Oxidschicht kann im einfachsten Fall in Form einer natürlichen Oxidschicht erfolgen, die entsteht, wenn die metallische Oberfläche des Drahtmetallkörpers der Umgebungsluft ausgesetzt wird.

Nach dem Umformen des Drahtes zu einem Drahtgebilde mit wenigstens einer Überkreuzung, d.h. zu einer Gitterstruktur bzw. einem Gittergeflecht, erfolgt die thermische Oxidierung im Salzbad. Dadurch wird die Oberfläche modifiziert, insbesondere passiviert und gehärtet. Da die natürlich ausgebildete Oxidschicht zumindest im oberflächennahen Grenzbereich nickelarm oder sogar nickelfrei ist und somit wie eine Barriere zur Metallgrenzfläche des Drahtes wirkt, enthält die thermische ausgebildete Oxidschicht ebenfalls nur einen geringen Ni-Gehalt bzw. ist zumindest im oberflächennahen Grenzbereich nickelarm oder sogar nickelfrei.

Durch die sich anschließende thermische Behandlung im Salzbad, insbesondere einem stickstoffhaltigen Salzbad, wird auf der natürlich ausgebildeten Oxidschicht eine dichte Mischoxidschicht erzeugt, die TiO₂ enthält. Zusätzlich enthält die Mischoxidschicht Anteile an Stickstoff, welcher als Titanoxinitrid und/oder Titanitrid gebunden ist. Titanoxinitrid und/oder Titanitrid ergibt sich aus dem Salzbad, bspw. bei einer Verwendung eines Alkalimetall-Stickstoff-Salzes, insbesondere Kaliumnitrat oder Natriumnitrit oder einem Gemisch aus Kaliumnitrat und Natriumnitrit, ergibt. Das thermisch ausgeformte Nitrid, insbesondere Titanoxinitrid und/oder Titaninitrid, wirkt als Hartstoff, der die Schichthärte erhöht und das Verschleiß- und Reibungsverhalten des Funktionselementes, insbesondere Implantats verbessert.

Die Erfindung ist nicht auf eine spezielle NiTi-Legierung eingeschränkt, sondern allgemein bei den in der Medizintechnik gebräuchlichen NiTi-Legierungen einsetzbar, aus denen intravaskuläre Funktionselemente, insbesondere Implantate hergestellt werden, deren Oberflächen durch eine Oxidschicht geschützt werden soll.

Als Legierung kommen bspw. verschiedene binäre Verbindungen auf Ni-Basis in Frage, wie bspw. NiTi Legierungen, insbesondere Nitinol (Ni 55 Gew.-%, Ti 45 Gew.-%), oder verschiedene ternäre Verbindungen wie bspw. NiTiFe oder NiTiNb oder NiTiCr oder quarternäre Legierungen wie NiTiCoCr.

Der Draht kann mindestens 5 Gew.-%, vorzugsweise mindestens 10 Gew.-%, vorzugsweise mindestens 20 Gew.-%, vorzugsweise mindestens 40 Gew.-%, Nickel aufweisen. Der Draht kann weiterhin vorzugsweise höchstens 80 Gew.-%, vorzugsweise höchstens 60 Gew.-%, vorzugsweise höchstens 55 Gew.-% vorzugsweise höchstens 50 Gew.-% Nickel aufweisen. Der Titangehalt kann vorzugsweise mindestens 10 Gew.-%, vorzugsweise mindestens 30 Gew.-%, vorzugsweise mindestens 40 Gew.-%, vorzugsweise mindestens 50 Gew.-%, betragen. Eine Obergrenze für den Titangehalt kann 90 Gew.-%, vorzugsweise 80 Gew.-%, vorzugsweise 65 Gew.-%, vorzugsweise 60 Gew.-%, vorzugsweise 55 Gew.-%, betragen.

Das Funktionselement, insbesondere Implantat ist bspw. ein geflochtener Stent oder ein anderes Implantat, beispielsweise ein Flow-Diverter oder ein Stent-Graft oder ein intravaskuläres Verschlussdevice oder ein intravaskuläres Coil.

Die Mischoxidschicht kann mindestens 10 Gew.-%, weiter vorzugsweise mindestens 20 Gew.-%, weiter vorzugsweise mindestens 30 Gew.-%, weiter vorzugsweise mindestens 40 Gew.-%, weiter vorzugsweise mindestens 50 Gew.%. Titanoxinitrid und/oder Titanitrid aufweisen.

Die Erfindung wird beispielhaft anhand eines intravaskulären Funktionselements in der Form eines Flow-Diverters erläutert, dessen Gitterstruktur bzw. Gittergeflecht aus mehreren Drähten hergestellt ist. Der Flow Diverter oder Strömungsteiler weist eine zumindest abschnittsweise, insbesondere vollständig rohrförmige Gitterstruktur auf. Die Gitterstruktur ist in radialer Richtung, d. h. senkrecht zur Längsachse der Gitterstruktur selbstexpandierbar. Die Gitterstruktur bzw. der die Gitterstruktur bildende Draht ist aus einem Formgedächtniswerkstoff hergestellt, konkret aus einer Legierung mit den Legierungselemente Nickel und Titan, insbesondere aus der unter dem Markennamen NITINOL bekannten Legierung. Die Oberfläche des Drahtes weist eine quadratische Rauheit R_{q} auf, die mindestens 0,01 µm beträgt (nicht erfindungsgemäß).

Die quadratische Rauheit R_{q} kann beispielsweise mindestens 0,0127 µm (0.5 µin) (nicht erfindungsgemäß); insbesondere mindestens 0,0254 µm (1 µin) betragen. Die maximale quadratische Rauheit R_{q} kann 0,08 µm, insbesondere 0,0762 µm (3 µin), insbesondere 0,0508 µm (1 µin) betragen.

Die vorstehend genannte quadratische Rauheit R_{q} kann durch die vorstehend genannte Oberflächenbehandlung des Drahtes bzw. der Drähte eingestellt werden.

Zusätzlich zu der vorstehend genannten Rauheit zeichnet sich das Funktionselement durch eine Mischoxidschicht auf der Oberfläche des Drahtes bzw. der Drähte aus, wobei die Schichtdicke der Mischoxidschicht mindestens 150 nm (nicht erfindungsgemäß), insbesondere mindestens 200 nm (nicht erfindungsgemäß) beträgt. Bei dem Beispiel nimmt der Sauerstoffgehalt nimmt bis zu einer Schichtdicke von ca. 25 nm ausgehend von der Oberfläche (0 nm) zu und bleibt bis ca. 50 nm konstant. Bis zu einer Schichttiefe von ca. 150 nm nimmt der Sauerstoffgehalt kontinuierlich ab. Umgekehrt steigt der NickelAnteil mit zunehmender Schichtdicke an und wird im Bereich von ca. 150 nm maximal. Ähnliches gilt für den Titan-Anteil. Im Bereich der Schichtdicke von ca. 100 nm überlagern sich die Sauerstoff-und Nickelkurven. In diesem Bereich bilden sich Mischoxide, wie TiOz und wenigstens ein Nitrid, insbesondere Titanoxinitrid und/oder Titanitrid.

Bei einem weiteren Beispiel beträgt die Dicke der Mischoxidschicht ca. 450 nm (nicht erfindungsgemäß).

Die maximale Dicke der Mischoxidschicht beträgt 450 nm (nicht erfindungsgemäß), insbesondere maximal 300 nm.

Die vorstehend genannte quadratische Rauheit R_{q} ermöglicht bzw. erleichtert die Ausbildung einer Mischoxidschicht der vorstehend genannten Dicke. Die Einstellung der vorstehend genannten Rauheit R_{q} durch mechanisches Polieren hat den weiteren Vorteil, dass der jeweilige Draht einen im Wesentlichen konstanten Drahtdurchmesser aufweist. Der Drahtdurchmesser weicht vom mittleren Drahtdurchmesser um maximal 10 %, insbesondere um maximal 5 %, insbesondere maximal 3 % ab. Der im Wesentlichen konstante Drahtdurchmesser führt dazu, dass die Drahteigenschaften, insbesondere die mechanischen Drahteigenschaften homogen entlang der gesamten Länge bzw. des gesamten Umfangs der Gitterstruktur vorliegen. Die homogenen mechanischen Eigenschaften erleichtern die Crimpbarkeit und verbessern das Verhalten des Funktionselements im Gefäß.

Der Draht ist aus einer binären NiTi-Legierung, wie Nitinol, hergestellt. Andere NiTi enthaltende Legierung sind möglich. Dabei wird die Modifikation der Oberfläche durch die thermische Behandlung im Salzbad dargestellt, die für die Einstellung der Stickstoffkonzentration in der TiOz Mischoxidschicht verantwortlich ist.

Das Grundelement des Funktionselementes, nämlich der Draht, wird im ersten Schritt oberflächenbehandelt, wobei die nach der Wärmebehandlung zur Konditionierung des Drahtes gebildete Oxidschicht entfernt wird. Auf dem oberflächenbehandelten Draht bildet sich durch Kontakt mit der Umgebungsluft spontan eine homogene natürliche Oxidschicht.

Im zweiten Schritt wird aus dem oberflächenbehandelten Draht die Gitterstruktur des Funktionselements, beispielsweise ein Stent, oder im Fall mehrerer oberflächenbehandelter Drähte ein Flow-Diverter geflochten. Im dritten Schritt wird das Funktionselement zur Erhöhung der Schichtdicke im Salzbad wärmebehandelt.

Das vorstehende Prinzip zur Herstellung des Funktionselements wird sowohl im Zusammenhang mit als auch allgemein, d.h. unabhängig von den konkreten Ausführungsbeispielen offenbart.

## Patentansprüche

1. Intravaskuläres Funktionselement, insbesondere Implantat, weiter insbesondere Stent, Flow-Diverter, Stent-Graft und intravaskuläres Verschlussdevice, mit einer zumindest abschnittsweise rohrförmigen, radial selbstexpandierbaren Gitterstruktur (12), die einen Draht (10) oder mehrere Drähte (10) aufweist, wobei der Draht (10) / wenigstens einer der Drähte (10) ein superelastisches Material, insbesondere ein superelastisches Material aus einer Legierung mit den Legierungselementen Nickel und Titan, umfasst,
**dadurch gekennzeichnet, dass**
die Mischoxidschicht auf der Oberfläche des Drahtes (10) / der Drähte (10) mit einer Schichtdicke von 250 nm bis 400 nm ausgebildet ist, wobei die quadratische Rauheit R_{q} des Drahtes (10) / der Drähte (10) von 0,02 µm bis 0,5 µm, insbesondere von 0,05 µm bis 0,2 µm, insbesondere von 0,06 µm bis 0,1 µm beträgt, und/oder die Rauheit, insbesondere die quadratische Rauheit R_{q}, in Umfangsrichtung des Drahtes (10) / der Drähte (10) größer als die Rauheit, insbesondere die quadratische Rauheit R_{q}, in Längsrichtung des Drahtes (10) / der Drähte (10) ist.

2. Funktionselement nach Anspruch 1
**dadurch gekennzeichnet, dass**
die Rauheit in Umfangsrichtung mindestens um den Faktor 1,5, insbesondere mindestens um den Faktor 2, insbesondere mindestens um den Faktor 5, insbesondere mindestens um den Faktor 10 größer als die Rauheit in Längsrichtung ist.

3. Funktionselement nach Anspruch 1 oder 2
**dadurch gekennzeichnet, dass**
die quadratische Rauheit R_{q} in Umfangsrichtung des Drahtes (10) / der Drähte (10) von 0,1 µm bis 0,5 µm beträgt und/oder die quadratische Rauheit R_{q} in Längsrichtung des Drahtes (10) / der Drähte (10) von 0,02 µm bis 0,1 µm, insbesondere von 0,05 µm bis 0,08 µm beträgt.

4. Funktionselement nach einem der vorhergehenden Ansprüche
**dadurch gekennzeichnet, dass**
der Durchmesser des Drahtes (10) / der Drähte (10) entlang der gesamten Drahtlänge im Wesentlichen konstant ist und insbesondere um höchstens 10%, insbesondere höchstens 5%, insbesondere höchstens 3%, insbesondere höchstens 2%, insbesondere höchstens 1% vom durchschnittlichen Durchmesser des Drahtes (10) / des jeweiligen Drahtes (10) abweicht und/ oder der durchschnittliche Durchmesser des Drahtes (10) / der Drähte (10) von 30 µm bis 60 µm, insbesondere von 40 µm bis 60 µm beträgt.

5. Funktionselement nach einem der vorhergehenden Ansprüche
**dadurch gekennzeichnet, dass**
der Nenndurchmesser der Gitterstruktur (12) von 3 mm bis 5,5 mm, insbesondere ca. 3,5 mm oder insbesondere ca. 4,5 mm beträgt.

6. Funktionselement nach einem der vorhergehenden Ansprüche
**dadurch gekennzeichnet, dass**
der Draht (10) / wenigstens einer der Drähte (10) ein röntgensichtbares Kernmaterial (11a) und ein superelastisches Mantelmaterial (11b) aufweist und/oder der Draht (10) / die Drähte (10) oberflächenbehandelt ist / sind, insbesondere deoxidiert, insbesondere mechanisch poliert und/oder chemisch poliert, insbesondere gebeizt.

7. Funktionselement nach einem der vorhergehenden Ansprüche
**dadurch gekennzeichnet, dass**
die Mischoxidschicht TiO₂ und wenigstens ein Nitrid, insbesondere Titanoxinitrid und/oder Titanitrid aufweist.

8. Funktionselement nach einem der vorhergehenden Ansprüche
**dadurch gekennzeichnet, dass**
die Gitterstruktur in Längsrichtung und in Umfangsrichtung jeweils Zellen aus sich kreuzenden Drähten (10) bildet und in Umfangsrichtung 16 Zellen bis 32 Zellen, insbesondere 20 Zellen bis 28 Zellen, insbesondere 24 Zellen aufweist, wobei die Gitterstruktur Schlaufen an einem einzigen axialen Ende aufweist.

9. Funktionselement nach Anspruch 11
**dadurch gekennzeichnet, dass**
der durchschnittliche Durchmesser der Drähte (10) jeweils von 35 µm bis 50 µm, insbesondere ca. 42 µm beträgt.

10. Funktionselement nach Anspruch 11 oder 12
**dadurch gekennzeichnet, dass**
das Kernmaterial (11a) Platin oder eine Platinlegierung umfasst und der Platinanteil von 10% bis 40%, insbesondere von 13% bis 35%, insbesondere von 20% bis 25% beträgt.

11. Funktionselement nach einem der Ansprüche 1 bis 10
**dadurch gekennzeichnet, dass**
- die Gitterstruktur in Längsrichtung und in Umfangsrichtung jeweils Zellen aus einem einzigen mit sich selbst verflochtenen Draht (10) bildet und in Umfangsrichtung 6 Zellen bis 16 Zellen, insbesondere 8 Zellen bis 12 Zellen aufweist, wobei die Gitterstruktur Schlaufen an beiden axialen Enden aufweist wobei
- insbesondere bei einem Nenndurchmesser der Gitterstruktur von 2,5 mm bis 3,5 mm der durchschnittliche Durchmesser des Drahtes (10) von 40 µm bis 55 µm, insbesondere zwischen 45 µm und 50 µm, beträgt und
- insbesondere bei einem Nenndurchmesser der Gitterstruktur von 3,5 mm bis 8 mm der durchschnittliche Durchmesser des Drahtes (10) von 45 µm bis 65 µm, insbesondere ca. 50 µm, beträgt.

12. Funktionselement nach Anspruch 10 oder 11
**dadurch gekennzeichnet, dass**
das Kernmaterial (11a) Platin oder eine Platinlegierung umfasst und der Platinanteil 20% bis 40%, insbesondere von 25% bis 35% beträgt und/oder ein Flechtwinkel α der Gitterstruktur (12) zwischen dem Draht (10) und einer in Längsrichtung der Gitterstruktur (12) verlaufenden Längsachse zumindest abschnittsweise zwischen 60° und 70°, insbesondere 65°, beträgt.

13. System umfassend ein Funktionselement nach einem der vorhergehenden Ansprüche sowie ein schlauchförmiges Element, insbesondere eine Schleuse, in der das Funktionselement angeordnet ist, und einen Transportdraht, an dem das Funktionselement befestigt ist, wobei ein Innendurchmesser des schlauchförmigen Elementes insbesondere höchstens 0,8 mm, weiter insbesondere höchstens 0,7 mm, weiter insbesondere höchstens 0,6 mm, weiter insbesondere höchstens 0,5 mm, weiter insbesondere höchstens 0,4 mm ist.

14. Verfahren zur Herstellung eines intravaskulären Funktionselementes nach Anspruch 1, das in ein Hohlorgan einführbar ist, bei dem
- vor der Herstellung einer Gitterstruktur (12) des Funktionselementes ein Draht (10) oder mehrere Drähte (10) oberflächenbehandelt werden, insbesondere deoxidiert, insbesondere mechanisch poliert und/oder chemisch poliert, insbesondere gebeizt werden, oder ein oberflächenbehandelter, insbesondere deoxidierter, insbesondere mechanisch polierter und/oder chemisch polierter, insbesondere gebeizter Draht (10) oder mehrere oberflächenbehandelte, insbesondere deoxidierte, insbesondere mechanische polierte und/oder chemisch polierte, insbesondere gebeizte Drähte (10) bereitgestellt werden,
- danach die Gitterstruktur (12) aus dem oberflächenbehandelten Draht (10) / den oberflächenbehandelten Drähten (10) gebildet, insbesondere geflochten wird und
- danach eine Mischoxidschicht auf die Oberfläche des Drahtes (10) / der Drähte (10) mit einer Schichtdicke von 250 nm bis 400 nm durch eine Wärmebehandlung in einem Salzbad aufgebracht wird.

15. Verfahren nach Anspruch 14
**dadurch gekennzeichnet, dass**
die Temperatur der Wärmebehandlung zwischen 450° und 600°, insbesondere zwischen 460° und 550°, insbesondere zwischen 480° und 540° beträgt und/oder die Gitterstruktur nach der Wärmebehandlung abgeschreckt, insbesondere in Wasser abgeschreckt wird.

## Claims

1. An intravascular functional element, in particular an implant, more particularly a stent, flow diverter, stent graft and intravascular occlusion device, with a radially self-expandable mesh structure (12) which is tubular at least in sections, which has a wire (10) or a plurality of wires (10), wherein the wire (10)/at least one of the wires (10) comprises a superelastic material, in particular a superelastic material produced from an alloy with nickel and titanium alloy elements,
**characterized in that**
the mixed oxide layer on the surface of the wire (10)/the wires (10) is formed with a layer thickness of 250 nm to 400 nm, wherein the quadratic roughness R_{q} of the wire (10)/the wires (10) is from 0.02 um to 0.5 um, in particular from 0.05 um to 0.2 µm, in particular from 0.06 µm to 0.1 µm, and/or the roughness, in particular the quadratic roughness R_{q}, in the circumferential direction of the wire (10)/the wires (10) is greater than the roughness, in particular the quadratic roughness R_{q}, in the longitudinal direction of the wire (10)/the wires (10) .

2. The functional element as claimed in claim 1,
**characterized in that**
the roughness in the circumferential direction is greater than the roughness in the longitudinal direction by a factor of at least 1.5, in particular by a factor of at least 2, in particular by a factor of at least 5, in particular by a factor of at least 10.

3. The functional element as claimed in claim 1 or claim 2,
**characterized in that**
the quadratic roughness R_{q} in the circumferential direction of the wire (10)/the wires (10) is from 0.1 um to 0.5 um and/or the quadratic roughness R_{q} in the longitudinal direction of the wire (10)/the wires (10) is from 0.02 um to 0.1 µm, in particular from 0.05 µm to 0.08 µm.

4. The functional element as claimed in one of the preceding claims,
**characterized in that**
the diameter of the wire (10)/the wires (10) is substantially constant along the entire length of the wire and in particular deviates by at most 10%, in particular at most 5%, in particular at most 3%, in particular at most 2%, in particular at most 1% from the mean diameter of the wire (10)/the respective wire (10) and/or the mean diameter of the wire (10)/the wires (10) is from 30 µm to 60 µm, in particular from 40 µm to 60 µm.

5. The functional element as claimed in one of the preceding claims,
**characterized in that**
the nominal diameter of the mesh structure (12) is from 3 mm to 5.5 mm, in particular approximately 3.5 mm or in particular approximately 4.5 mm.

6. The functional element as claimed in one of the preceding claims,
**characterized in that**
the wire (10)/at least one of the wires (10) has an X-ray visible core material (11a) and a superelastic sheath material (11b) and/or the wire (10)/the wires (10) is/are surface treated, in particular deoxidized, in particular mechanically polished and/or chemically polished, in particular pickled.

7. The functional element as claimed in one of the preceding claims,
**characterized in that**
the mixed oxide layer contains TiO₂ and at least one nitride, in particular titanium oxynitride and/or titanium nitride.

8. The functional element as claimed in one of the preceding claims,
**characterized in that**
in the longitudinal direction and in the circumferential direction, the mesh structure respectively forms cells of intersecting wires (10) and in the circumferential direction, it has 16 cells to 32 cells, in particular 20 cells to 28 cells, in particular 24 cells, wherein the mesh structure has loops on a single axial end.

9. The functional element as claimed in claim 11,
**characterized in that**
the mean diameter of the wires (10) is respectively from 35 um to 50 um, in particular approximately 42 µm.

10. The functional element as claimed in claim 11 or claim 12,
**characterized in that**
the core material (11a) comprises platinum or a platinum alloy, and the proportion of platinum is from 10% to 40%, in particular from 13% to 35%, in particular from 20% to 25%.

11. The functional element as claimed in one of claims 1 to 10,
**characterized in that**
- in the longitudinal direction and in the circumferential direction, the mesh structure respectively forms cells produced from a single wire (10) interwoven with itself, and in the circumferential direction, it has 6 cells to 16 cells in particular 8 cells to 12 cells, wherein the mesh structure has loops at both axial ends, wherein
- in particular in the case of a nominal diameter for the mesh structure of 2.5 mm to 3.5 mm, the mean diameter of the wire (10) is from 40 µm to 55 µm, in particular between 45 µm and 50 µm, and
- in particular in the case of a nominal diameter for the mesh structure of 3.5 mm to 8 mm, the mean diameter of the wire (10) is from 45 µm to 65 µm, in particular approximately 50 µm.

12. The functional element as claimed in claim 10 or claim 11,
**characterized in that**
the core material (11a) comprises platinum or a platinum alloy, and the proportion of platinum is 20% to 40%, in particular from 25% to 35% and/or a braiding angle α of the mesh structure (12) between the wire (10) and a longitudinal axis extending in the longitudinal direction of the mesh structure (12) is between 60° and 70°, in particular 65°, at least in sections.

13. A system comprising a functional element as claimed in one of the preceding claims as well as a tubular element, in particular a connector, in which the functional element is disposed, and a transport wire to which the functional element is attached, wherein an internal diameter of the tubular element is in particular at most 0.8 mm, more particularly at most 0.7 mm, even more particularly at most 0.6 mm, yet more particularly at most 0.5 mm, yet more particularly at most 0.4 mm.

14. A method for the production of an intravascular functional element as claimed in claim 1, which can be inserted into a hollow organ, in which
- prior to the production of a mesh structure (12) of the functional element, a wire (10) or a plurality of wires (10) are surface-treated, in particular deoxidized, in particular mechanically polished and/or chemically polished, in particular pickled, or a surface-treated, in particular deoxidized, in particular mechanically polished and/or chemically polished, in particular pickled wire (10) or a plurality of surface-treated, in particular deoxidized, in particular mechanically polished and/or chemically polished, in particular pickled wires (10) are provided,
- thereafter, the mesh structure (12) is formed, in particular braided, from the surface-treated wire (10)/the surface-treated wires (10), and
- thereafter, a mixed oxide layer is applied to the surface of the wire (10)/the wires (10) with a layer thickness of 250 nm to 400 nm by means of a heat treatment in a salt bath.

15. The method as claimed in claim 14,
**characterized in that**
the temperature of the heat treatment is between 450° and 600°, in particular between 460° and 550°, in particular between 480° and 540° and/or after the heat treatment, the mesh structure is quenched, in particular quenched in water.

## Revendications

1. Élément fonctionnel intravasculaire, en particulier implant, plus particulièrement stent, déviateur de flux, endoprothèse et dispositif obturateur intravasculaire, doté d'une structure grillagée au moins de forme tubulaire par sections et auto-expansible radialement (12), qui présente un fil (10) ou plusieurs fils (10), le fil (10)/au moins un des fils (10) comprenant un matériau super-élastique, en particulier un matériau super-élastique composé d'un alliage contenant les éléments d'alliage nickel et titane,
**caractérisé en ce que**
la couche d'oxyde mixte est constituée sur la surface du fil (10)/des fils (10) avec une épaisseur de couche de 250 nm à 400 nm, la rugosité quadratique R_{q} du fil (10)/des fils (10) étant de 0,02 µm à 0,5 µm, en particulier de 0,05 µm à 0,2 µm, en particulier de 0,06 µm à 0,1 µm, et/ou la rugosité, en particulier la rugosité quadratique R_{q}, étant, dans le sens circonférentiel du fil (10)/des fils (10), supérieure à la rugosité, en particulier la rugosité quadratique R_{q}, dans le sens longitudinal du fil (10)/des fils (10) .

2. Élément fonctionnel selon la revendication 1,
**caractérisé en ce que**
la rugosité dans le sens circonférentiel est supérieure à la rugosité dans le sens longitudinal au moins à raison du facteur 1,5, en particulier au moins à raison du facteur 2, en particulier au moins à raison du facteur 5, en particulier au moins à raison du facteur 10.

3. Élément fonctionnel selon la revendication 1 ou 2,
**caractérisé en ce que**
la rugosité quadratique R_{q} dans le sens circonférentiel du fil (10)/des fils (10) est de 0,1 µm à 0,5 µm et/ou la rugosité R_{q} dans le sens longitudinal du fil (10)/des fils (10) est de 0,02 µm à 0,1 µm, en particulier de 0,05 µm à 0,08 µm.

4. Élément fonctionnel selon une des revendications précédentes,
**caractérisé en ce que**
le diamètre du fil (10)/des fils (10) le long de toute la longueur du fil est sensiblement constant et diffère en particulier d'au plus 10 %, en particulier d'au plus 5 %, en particulier d'au plus 3 %, en particulier d'au plus 2 %, en particulier d'au plus 1 % du diamètre moyen du fil (10)/du fil respectif (10) et/ou le diamètre moyen du fil (10)/des fils (10) est de 30 µm à 60 µm, en particulier de 40 µm à 60 µm.

5. Élément fonctionnel selon une des revendications précédentes,
**caractérisé en ce que**
le diamètre nominal de la structure grillagée (12) est de 3 mm à 5,5 mm, en particulier environ 3,5 mm ou en particulier environ 4,5 mm.

6. Élément fonctionnel selon une des revendications précédentes,
**caractérisé en ce que**
le fil (10)/au moins un des fils (10) présente un matériau de noyau (11a) visible par radiographie et un matériau de chemise super-élastique (11b) et/ou que le fil (10)/les fils (10) est/sont traité(s) en surface, en particulier désoxydé(s), en particulier poli(s) mécaniquement et/ou poli(s) chimiquement, en particulier décapé(s).

7. Élément fonctionnel selon une des revendications précédentes,
**caractérisé en ce que**
la couche d'oxyde mixte présente du TiO₂, et au moins un nitrure, en particulier du nitrure d'oxyde de titane et/ou du nitrure de titane.

8. Élément fonctionnel selon une des revendications précédentes,
**caractérisé en ce que**
la structure grillagée forme, dans le sens longitudinal et dans le sens circonférentiel, respectivement des cellules composées de fils se croisant (10) et, dans le sens circonférentiel, 16 cellules à 32 cellules, en particulier 20 cellules à 28 cellules, en particulier 24 cellules, la structure grillagée présentant des boucles à une seule extrémité axiale.

9. Élément fonctionnel selon la revendication 11,
**caractérisé en ce que**
le diamètre moyen des fils (10) est respectivement de 35 µm à 50 µm, en particulier environ 42 µm.

10. Élément fonctionnel selon la revendication 11 ou 12,
**caractérisé en ce que**
le matériau de noyau (11a) comprend du platine ou un alliage de platine et que la teneur en platine est de 10 % à 40 %, en particulier 13 % à 35 %, en particulier 20 % à 25 %.

11. Élément fonctionnel selon une des revendications 1 à 10,
**caractérisé en ce que**
- la structure grillagée constitue, dans le sens longitudinal et dans le sens circonférentiel, respectivement des cellules composées d'un seul fil auto-entrelacé (10) et présente dans le sens circonférentiel 6 cellules à 16 cellules, en particulier 8 cellules à 12 cellules, la structure grillagée présentant des boucles à ses deux extrémités axiales,
- en particulier en cas d'un diamètre nominal de la structure grillagée de 2,5 mm à 3,5 mm, le diamètre moyen du fil (10) étant de 40 µm à 55 µm, en particulier de 45 µm à 50 µm, et
- en particulier dans le cas d'un diamètre nominal de la structure grillagée de 3,5 mm à 8 mm, le diamètre moyen du fil (10) étant de 45 µm à 65 µm, en particulier d'environ 50 µm.

12. Élément fonctionnel selon la revendication 10 ou 11,
**caractérisé en ce que**
le matériau de noyau (11a) comprend du platine ou un alliage de platine et la teneur en platine est de 20 % à 40 %, en particulier 25 % à 35 % et/ou un angle de tressage α de la structure grillagée (12), entre le fil (10) et un axe longitudinal s'étendant dans le sens longitudinal de la structure grillagée (12), étant du moins par sections de 60° à 70°, en particulier 65°.

13. Système comprenant un élément fonctionnel selon une des revendications précédentes et un élément de forme tubulaire, en particulier un sas, dans lequel l'élément fonctionnel est disposé, et un câble de transport auquel l'élément fonctionnel est fixé, un diamètre intérieur de l'élément de forme tubulaire étant en particulier au plus de 0,8 mm, plus particulièrement au plus de 0,7 mm, plus particulièrement au plus de 0,6 mm, plus particulièrement au plus de 0,5 mm, plus particulièrement au plus de 0,4 mm.

14. Procédé de fabrication d'un élément fonctionnel intravasculaire selon la revendication 1, qui est introductible dans un organe creux, dans lequel
- avant la fabrication d'une structure grillagée (12) de l'élément fonctionnel, un fil (10) ou plusieurs fils (10) sont traités en surface, en particulier désoxydés, en particulier polis mécaniquement et/ou polis chimiquement, en particulier décapés, ou un fil (10) traité en surface, en particulier désoxydé, en particulier poli mécaniquement et/ou poli chimiquement, en particulier décapé ou plusieurs fils (10) traités en surface, en particulier désoxydés, en particulier polis mécaniquement et/ou polis chimiquement, en particulier décapés sont mis à disposition,
- la structure grillagée (12) est ensuite formée, en particulier tressée, à partir du fil (10) traité en surface/des fils (10) traités en surface et
- une couche d'oxyde mixte est ensuite appliquée sur la surface du fil (10)/des fils (10) en une épaisseur de couche de 250 nm à 400 nm par un traitement thermique dans un bain salé.

15. Procédé selon la revendication 14,
**caractérisé en ce que**
la température du traitement thermique est de 450° à 600°, en particulier de 460° à 550°, en particulier de 480° à 540° et/ou la structure grillagée est trempée, en particulier trempée dans de l'eau après le traitement thermique.
